# EUROPEAN PATENT APPLICATION

(11) **EP 4 223 291 A1**
(43) Date of publication of application: **09.08.2023**
(21) Application number: 21852860.2
(22) Date of filing: 29.07.2021
(51) Int. Cl.: A61K 31/325, A61P 43/00, A61P 1/16, A61P 11/00, A61P 37/00, A61P 35/00

(54) **COMPOSITION FOR TREATING KCA3.1 CHANNEL-MEDIATED DISEASES COMPRISING PHENYLALKYL CARBAMATE COMPOUND**

(30) Priority: 03.08.2020 KR 20200096595
(71) Applicant: Cellionbiomed Inc., Daejeon 34013 (KR)
(72) Inventor: KIM, Seong-Jin, Sejong 30062 (KR); SUH, Suk-Hyo, Gyeonggi-do 10323 (KR)
(74) Representative: BCKIP Part mbB
(86) International application number: PCT/KR2021/009884
(87) International publication number: WO 2022/030879

(57) **Abstract**

The present invention relates to a composition for treating K_{Ca}3.1 channel-mediated diseases, comprising a phenylalkyl carbamate compound and, more specifically, to a pharmaceutical composition comprising, as an active ingredient, a phenylalkyl carbamate compound, the representative example of which is solriamfetol conventionally used as a therapeutic agent for narcolepsy, i.e., 2-amino-3-phenylpropyl carbamate, the pharmaceutical composition being able to be used for treating K_{Ca}3.1 channel-mediated diseases, for example, fibrotic diseases, autoimmune diseases, and cancer diseases, by inhibiting the activation of K_{Ca}3.1 channels in the cell membrane.

## Description

### [Technical Field]

The present invention relates to a composition for treating a K_{Ca}3.1 channel-mediated disease, including a phenylalkyl carbamate compound, and more particularly, to a pharmaceutical composition which includes a phenylalkyl carbamate compound represented by solriamfetol, i.e., 2-amino-3-phenylpropyl carbamate, conventionally used as a therapeutic agent for narcolepsy, as an active ingredient, and can be used for treatment of K_{Ca}3.1 channel-mediated diseases, such as fibrotic diseases, autoimmune diseases, and cancer diseases, by inhibiting the expression of a K_{Ca}3.1 channel in the cell membrane.

### [Background Art]

K_{Ca}3.1 channels in the human body are distributed in non-excitable cells, including fibroblasts, hepatic stellate cells, vascular endothelial cells, nerve cells, cancer cells, and immune cells (T cells and B cells). Generally, a K⁺ channel serves to activate cells by increasing the influx of Ca²⁺ into cells through membrane voltage hyperpolarization. However, Ca²⁺ acts as a second messenger or a co-factor for various enzymes in cells, and plays a very important role in the intracellular signal transduction process. Therefore, when a K_{Ca}3.1 channel is activated and thus Ca²⁺ in cells increases, cell proliferation, epithelial-mesenchymal transition, cell migration, the extracellular matrix, or the production and secretion of a material such as nitrogen oxide are promoted.

The formation of myofibroblasts (or activated hepatic stellate cells) is the most important step in the fibrosis process and occurs due to epithelial-mesenchymal transition. When myofibroblasts are formed and hepatic stellate cells are activated, the proliferation of these cells actively occurs, and fibrosis actively occurs due to extracellular matrix formation in the cells. The epithelial-mesenchymal transition in which myofibroblasts are formed from vascular endothelial cells or epithelial cells is known to be a Ca²⁺-dependent process. The epithelial-mesenchymal transition caused by TGF_{β}, which is a pro-fibrotic agent, is known to be particularly sensitive to Ca²⁺. K_{Ca}3.1 channels control intracellular Ca²⁺ in myofibroblasts and hepatic stellate cells and control epithelial-mesenchymal transition.

When a K_{Ca}3.1 channel is activated, different responses occur according to cells expressing the K⁺ channel. Through immune cells, inflammatory and immune responses are increased through the proliferation of immune cells and the secretion of cytokines. It promotes angiogenesis using vascular endothelial cells and induces vasodilation due to the secretion of nitric oxide. In addition, a fibrotic disease is induced by fibroblasts and hepatic stellate cells. That is, it generates myofibroblasts or activates hepatic stellate cells, and induces the extracellular matrix such as collagen, thereby contributing to the formation of connective tissue. In addition, it also plays an important role in the proliferation and metastasis of some cancer cells. Therefore, the K_{Ca}3.1 channel may be assumed to play a very important role in the progression of inflammatory and autoimmune diseases, fibrotic diseases, and cancer diseases.

Pro-inflammatory agents such as cytokines and H₂O₂ amplify immune and inflammatory responses such as the proliferation of immune cells, and pro-fibrotic agents including growth factors such as TGF_{β} and PDGF amplify a fibrotic response. However, such pro-inflammatory agents and pro-fibrotic agents increase the expression of the K_{Ca}3.1 channel. That is, the increase in expression of the K_{Ca}3.1 channel plays an important role in the process of amplifying inflammatory and immune responses and a fibrotic response by pro-inflammatory agents and pro-fibrotic agents.

As such, when the expression of the K_{Ca}3.1 channel increases, the progression of proliferative diseases such as fibrotic and cancer diseases, and inflammatory diseases such as autoimmune diseases is promoted. Therefore, such diseases may be defined as K_{Ca}3.1 channel-mediated diseases. Recently, efforts to develop therapeutic agents for inflammatory and autoimmune diseases, fibrotic diseases, and cancer using a K_{Ca}3.1 channel-inhibitor drug are continuously being attempted. As part of these efforts, senicapoc, which is a representative K_{Ca}3.1 inhibitor, is being developed as a therapeutic agent for various types of inflammatory and autoimmune diseases and fibrotic diseases, including sickle cell anemia.

In US Patent No. US 9,259,412 B2 and its Family patent, Korean Patent No. 10-1414831, the inventors (Ewha University-Industry Collaboration Foundation) have suggested a composition for treating or preventing a K_{Ca}3.1 channel-mediated disease, including modafinil or a derivative thereof as an active ingredient, which has been used as a therapeutic agent for narcolepsy. Here, examples of such K_{Ca}3.1 channel-mediated diseases include sickle cell anemia, immune diseases such as acute immune responses and autoimmune diseases, cancer diseases such as prostate or pancreatic cancer, traumatic brain injury, cerebral ischemia, and secretory diarrhea.

Meanwhile, solriamfetol, i.e., 2-amino-3-phenylpropyl carbamate, is a therapeutic agent for narcolepsy, and is developed and sold under the product name 'Sunosi' by SK Corporation in Korea. In this regard, in US Patent Nos. US 5,955,499 B2 and US 6,140,532 B2 and their Family patent, Korean Patent Nos. 10-197892 and 10-173863 of SK Corporation, it is reported that solriamfetol or a derivative thereof is useful as a therapeutic agent for the central nervous system, particularly, an antidepressant and an antianxiety drug.

In addition, in US Patent No. US 9,464,041 B2 and its family patent, Korean Unexamined Patent Application Publication No. 10-2019-105675, a method of treating or preventing fatigue associated with diseases such as depression, cancer, multiple sclerosis, Parkinson's disease, Alzheimer's disease, chronic fatigue, fibromyalgia, chronic pain, traumatic brain injury, AIDS, and osteoarthritis using solriamfetol or a derivative thereof is disclosed, and in US Patent No. US 10,351,517 B2 and its family patent, Korean Patent No. 10-1335941, a method of treating sleep-wake disorders including excessive daytime sleepiness and pathological somnolence is disclosed.

### [Related Art Documents]

### [Patent Documents]

US Patent No. US 9,259,412 B2 (Korean Patent No. 10-1414831)
US Patent No. US 5,955,499 B2 (Korean Patent No. 10-197892)
US Patent No. US 6,140,532 B2 (Korean Patent No. 10-173863)
US Patent No. US 9,464,041 B2 (Korean Unexamined Patent Application Publication No. 10-2019-105675)
US Patent No. US 10,351,517 B2 (Korean Patent No. 10-1335941)

### [Disclosure]

### [Technical Problem]

While studying a method of treating a K_{Ca}3.1 channel-mediated disease using a material for inhibiting a K⁺ channel, the present inventors surprisingly identified the possibility that phenylalkyl carbamate compounds including solriamfetol conventionally used as a therapeutic agent for narcolepsy can inhibit the expression of the K_{Ca}3.1 channel, and proved this possibility through various experiments, and thus the present invention was completed.

The present invention is directed to providing a novel composition for treating a K_{Ca}3.1 channel-mediated disease, including the phenylalkyl carbamate compound as an active ingredient.

### [Technical Solution]

The present invention provides a composition for treating a K_{Ca}3.1 channel-mediated disease according to the present invention including a phenylalkyl carbamate compound represented by Chemical Formula 1 below or a pharmaceutically acceptable salt thereof as an active ingredient.

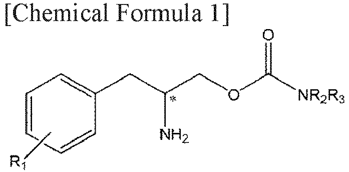

In Chemical Formula 1, R₁ is one or two functional groups selected from hydrogen, halogen, hydroxyl, amine, nitro, hydrogen sulfide, methyl, methylhalogen, ethyl, propyl, methoxy, ethoxy, vinyl and aryl, each of R₂ and R₃ is one functional group selected from hydrogen, methyl, ethyl, propyl, and amide, and ^{∗} indicates a chiral center.

In addition, the composition for treating a K_{Ca}3.1 channel-mediated disease according to the present invention includes a compound in which R₂ and R₃ of Chemical Formula 1 are hydrogen.

In addition, the composition for treating a K_{Ca}3.1 channel-mediated disease according to the present invention includes a compound in which R₁ is one or two functional groups selected from hydrogen, F, Cl, Br, and I in Chemical Formula 1.

In addition, the composition for treating a K_{Ca}3.1 channel-mediated disease according to the present invention includes a compound in which, in Chemical Formula 1, R₁ is one or two Fs, and each of R₂ and R₃ is one selected from hydrogen, methyl and amide.

In addition, the compound represented by Chemical Formula 1 is a chiral compound having an R-isomer or S-isomer content of 90% or more.

In addition, the compound represented by Chemical Formula 1 is any one selected from 2-amino-3-phenylpropylcarbamate; 2-amino-3-(3-fluorophenyl)propylcarbamate; 2-amino-3-(3,4-dichlorophenyl)propylcarbamate; 2-amino-3-phenylpropylmethylcarbamate; 2-amino-3-phenylpropyl(aminocarbonyl)carbamate; 2-amino-3-(4-hydroxylphenyl)propylcarbamate; and 2-amino-3-[3-(trifluoromethyl)phenyl]propylcarbamate.

In addition, the K_{Ca}3.1 channel-mediated disease is a fibrotic disease such as liver fibrosis or lung fibrosis, an autoimmune disease, or a cancer disease.

### [Advantageous Effects]

It was confirmed that a compound of Chemical Formula 1 according to the present invention has an effect of inhibiting inflammation and fibrosis as well as an effect of inhibiting K_{Ca}3.1 channel expression in an *ex vivo* experiment on fibroblasts, and also has an effect of inhibiting inflammation and fibrosis in an *in vivo* experiment on liver disease-induced mouse models.

Accordingly, the compound of Chemical Formula 1 can be effectively used as a novel pharmaceutical composition for treating various types of inflammatory and autoimmune diseases, such as a K_{Ca}3.1 channel-mediated disease occurring in the human body, fibrotic diseases, and cancer diseases, and as needed, is expected to be developed as a drug for animals.

### [Description of Drawings]

FIG. 1 shows the effect of a compound of Chemical Formula 2 according to the present invention on K_{Ca}3.1 current in fibroblasts.
FIG. 2 shows the effect of compounds of Chemical Formulas 3 to 8 according to the present invention on K_{Ca}3.1 current in fibroblasts.
FIG. 3 shows the effect of the compounds of Chemical Formulas 2 to 8 according to the present invention on the expression of an inflammatory marker in fibroblasts exposed to lipopolysaccharides (LPS), which cause inflammation, for 24 hours.
FIG. 4 shows the effect of the compounds of Chemical Formulas 2 to 8 according to the present invention on the expression of a fibrotic marker in fibroblasts exposed to TGF_{β} , which causes fibrosis, for 24 hours.
FIG. 5 shows the result of confirming the fibrosis inhibitory effect of a compound of Chemical Formula 2 according to the present invention in a mouse model in which lung fibrosis is induced by bleomycin through Masson's trichrome staining for collagen.
FIG. 6 shows the effect of a compound of Chemical Formula 2 according to the present invention on the expression of fibrotic marker mRNA in a mouse model in which lung fibrosis is induced by bleomycin.
FIG. 7 shows the result of confirming the fibrosis inhibitory effect of a compound of Chemical Formula 2 according to the present invention in a mouse model in which liver fibrosis is induced by a CDAHFD diet through Masson's trichrome staining for collagen.
FIG. 8 shows the result of the effect of a compound of Chemical Formula 2 according to the present invention on the expression of fibrotic marker mRNA in a mouse model in which liver fibrosis is induced by a CDAHFD diet.

### [Modes of the Invention]

The phenylalkylcarbamate compound of Chemical Formula 1 according to the present invention specifically includes compounds of Chemical Formulas 2 to 8 below.

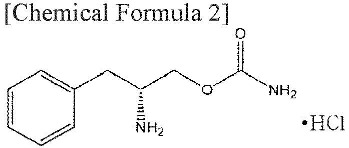

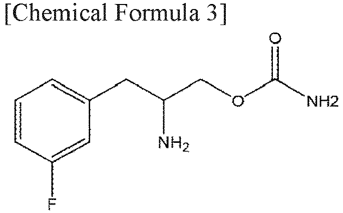

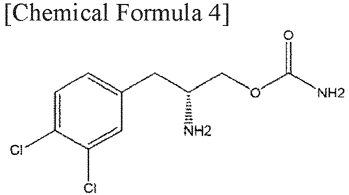

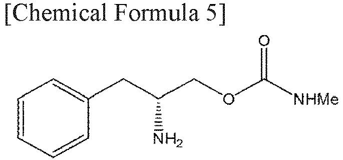

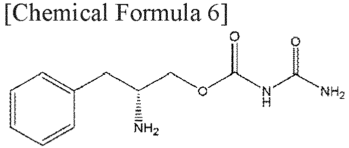

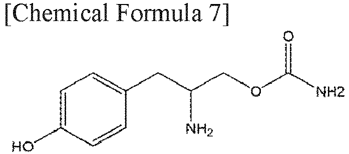

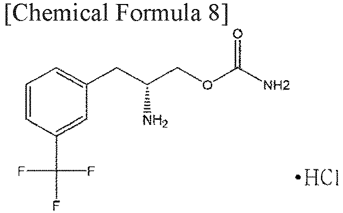

The chemical names and code names given to the compounds of Chemical Formulas 2 to 8 by the present inventors are as follows.

**Table 1**

| Chemical Formula | Chemical name | Code name |
|---|---|---|
| 2 | (R)-2-amino-3-phenyl propylcarbamate hydrochloride | SF-2 |
| 3 | 2-amino-3-(3-fluorophenyl)propylcarbamate | SF-3 |
| 4 | (2R)-2-amino-3-(3,4-dichlorophenyl)propylcarbamate | SF-4 |
| 5 | (2R)-2-amino-3-phenylpropyl methylcarbamate | SF-5 |
| 6 | (2R)-2-amino-3-phenylpropyl(aminocarbonyl)carbamate | SF-6 |
| 7 | 2-amino-3-(4-hydroxyphenyl)propylcarbamate | SF-7 |
| 8 | (2R)-2-amino-3-[3-(trifluoromethyl)phenyl]propylcarbamate hydrochloride | SF-8 |

The compound of Chemical Formula 2 is known under the general name solriamfetol, and is currently used as a therapeutic agent for narcolepsy. A method of preparing the compound of Chemical Formula 3 is disclosed in US Patent No. US 6,140,532 B2 and its family patent, Korean Patent No. 10-173863, and the compound of Chemical Formula 4 may be prepared by the method disclosed in US Unexamined Patent Application Publication No. US 2005/0080268 A1.

A method of preparing the compound of Chemical Formula 5 is disclosed in US Patent No. US 5,705,640 B2, and a method of preparing the compound of Chemical Formula 6 is disclosed in US Patent No. US 9,403,761 B2 or its family patent, Korean Unexamined Patent Application Publication No. 10-2016-0126988, and the compound of Chemical Formula 7 may be prepared by the method disclosed in Example 9 in US Patent No. US 6,140,532 B2 and Example 9 in International Publication No. WO 98/15526.

The compound of Chemical Formula 8 may be prepared by the method disclosed in Example 48A in US Patent No. US 9,180,120 B2 filed by Bayer or its family patent, Korena Unexamined Patent Application Publication No. 10-2013-0138216.

For reference, the Bayer patent discloses that the compound of Chemical Formula 8 can be used for liver cirrhosis. However, in the Bayer patent, the compound of Chemical Formula 8, as a V1a, V2 receptor antagonist, is for treating heart failure by inhibiting the action of vasopressin, which is an antidiuretic hormone, and treating liver cirrhosis including cardiovascular disorders through renal and hemodynamic effects.

On the other hand, in the present invention, the compound of Chemical Formula 8 is intended to solve the direct cause of fibrosis by inhibiting K_{Ca}3.1 channel expression, and the present invention has a clear difference from the cirrhosis treatment incidentally obtained by the Bayer patent.

A pharmaceutical composition according to the present invention includes a pharmaceutically acceptable salt of the compound of Chemical Formula 1. Here, the "pharmaceutically acceptable salt" may typically include a metal salt, a salt with an organic base, a salt with an inorganic acid, a salt with an organic acid, and a salt with a basic or acidic amino acid.

In addition, the pharmaceutical composition according to the present invention may include both solvates and hydrates of the compound of Chemical Formula 1, racemates, all possible stereoisomers thereof, and further include a crystalline or amorphous form of each compound.

The pharmaceutical composition according to the present invention may be formulated in the form of tablets, pills, powder, granules, capsules, a suspension, a liquid for internal use, an emulsion, a syrup, an aerosol, or a sterile injectable solution. In addition, the pharmaceutical composition of the present invention may be administered orally or parenterally depending on the purpose of use, and when parenterally administered, may be administered by topical application, intraperitoneal injection, intrarectal injection, subcutaneous injection, intravenous injection, intramuscular injection, or intrathoracic injection.

A dose of the pharmaceutical composition according to the present invention may vary according to a patient's weight, age and sex, a health condition, a diet, administration time, an administration method, an excretion rate and the severity of a disease. A daily dose is preferably 0.2 to 20 mg/kg, and more preferably 0.5 to 10 mg/kg based on the active ingredient, and the pharmaceutical composition according to the present invention may be administered once or twice daily, but the present invention is not limited thereto.

Hereinafter, the pharmacological effects of the phenylalkylcarbamate compound according to the present invention will be described.

### 1) Experimental method

### 1-1) Fibroblast culture

Fibroblasts (CRL-2795; American Type Culture Collection, VA) were cultured in Dulbecco's Modified Eagle Medium (Hyclone, Logan, UT). All cells were maintained in 5% CO₂ under a humid condition at 37 °C. The cultured fibroblasts were exposed to pro-inflammatory agents such as lipopolysaccharides (LPS) and a pro-fibrotic agent TGF_{β}, and the compounds of Chemical Formulas 2 to 8 of the present invention (hereinafter, referred to as SF-2 to SF-8 compounds) for 24 hours, and then anti-inflammatory and anti-fibrotic effects were tested.

### 1-2) Construction of lung fibrosis mouse model

To confirm the effect of the SF-2 compound according to the present invention on the inhibition of fibrosis, an experiment was performed as follows. First, C57BL/6 wild-type mice (purchased from Orient Bio) were divided into three groups of 6 to 8, and used as a disease-induced group, a drug-administered group, and a normal control. The test mice for each group were treated as follows.
(1) Disease-induced group: Lung fibrosis was induced by injecting 1.5 units of bleomycin into the airways of test mice. In addition, the same amount of distilled water as the SF-2 compound administered to the following drug-administered group was intraperitoneally injected (100 mg/kg) 5 times a week. In the accompanying drawing, bleomycin (BLM) refers to this disease-induced group.
(2) Drug-administered group: 1.5 units of bleomycin was injected into the airways of test mice, and then the SF-2 compound of the present invention was intraperitoneally injected (100 mg/kg) five times a week. In the accompanying drawing, BLM+SF-2 indicates the drug-administered group.
(3) Normal control: The same amount of distilled water as that of bleomycin injected into the disease-induced group was injected into the airways. In addition, the same amount of distilled water as that of the SF-2 compound administered to the drug-administered group was intraperitoneally injected five times a week. In the accompanying drawing, C or Control indicates the normal group.

The mouse models for each group were treated with drugs for 4 weeks in the same way as described above and immediately killed by administering an excess of an anesthetic, and then the lungs were extracted and used in the subsequent test.

### 1-3) Construction of fatty liver disease mouse model

To confirm the therapeutic effect of the SF-2 compound of the present invention on liver inflammation and fibrosis, an experiment was performed as follows. First, C57BL/6 wild-type mice (purchased from Orient Bio) were divided into three groups of 6 to 8, and used as a disease-induced group, a drug-administered group, and a normal control. The test mice for each group were treated as follows.
(1) Drug-induced group: Test mice were fed a CDAHFD diet (choline deficient, L-amino-acid-defined, high-fat diet with 0.1% methionine. A06071302, Research Diets, New Brunswick, NJ) to induce fatty liver disease and fibrosis. In addition, the same amount of distilled water as that of the SF-2 compound administered to the following drug-administered group five times a week using a zonde (oral tube). In the accompanying drawing, CDAHFD indicates the disease-induced group.
(2) Drug-administered group: The SF-2 compound of the present invention, together with the CDAHFD diet, was administered (100mg/kg/day) to test mice five times a week. In the accompanying drawing, CDAHFD+SF-2 indicates the drug-administered group.
(3) Normal control: Test mice were fed a normal diet. In addition, the same amount of distilled water as that of the SF-2 compound was administered to the drug-administered group five times a week using a zonde (oral tube). In the accompanying drawing, C or Control indicates the normal group.

The mouse models for each group were treated with drugs for 16 weeks in the same way as described above and immediately killed by administering an excess of an anesthetic, and then the livers were extracted and used in the subsequent test.

### 1-4) Preparation of paraffinized tissue specimens of lung and liver tissues and observation of morphological changes thereof

The lung and liver tissues extracted from the mouse model were fixed with a paraformaldehyde solution, and cut to a thickness of 1 to 2 mm. The cut tissue was embedded in paraffin and cut to a thickness of 4 µm, the paraffin was removed with xylene, the xylene was removed with ethanol, and the resultant was washed with tap water, thereby obtaining a paraffinized tissue specimen.

Tissue immunohistochemistry for collagen, which is a fibrotic marker, in the lung and liver tissues, was performed by Masson's trichrome staining.

### 1-5) Real time PCR analysis

The degrees of mRNA expression of pro-inflammatory or pro-fibrotic agents in lung and liver tissues extracted from the mouse models were measured by real time PCR. RNAs of these tissues were isolated using a TRIzol reagent (Molecular Research Center, Cincinnati, OH), and single-stranded cDNA was synthesized using BcaBEST polymerase (Takara Shuzo), followed by performing PCR.

Primer sequences (SEQ ID NOs: 1 to 30) of the pro-inflammatory cytokines and fibrotic markers used herein are shown in Tables 2 and 3 below.

**[Table 2]**

| | Pro-inflammatory cytokine primer sequences | | | |
|---|---|---|---|---|
| | Sense | SEQ ID NO: | Anti-sense | SEQ ID NO: |
| TNFα | F-CCCCAAAGGGATGAGAAGTT | 5 | R-CACTTGGTGGTTTGCTACGA | 6 |
| CCL2 | F-CCCCAAGAAGGAATGGGTCC | 7 | R-TGCTTGAGGTGTTTGTGGAA | 8 |
| IL1α | F-GAGCCGGGTGACAGTATCAG | 11 | R-ACTTCTGCCTGACGAGCTTC | 12 |
| IL6 | F-ACCAGAGGAAATTTTCAATA GGC | 13 | R-TGATGCACTTGCAGAAAACA | 14 |
| mGAPDH | F-CCGTATTGGGCGCCTGGTCA | 19 | R-CCGGCCTTCTCCATGGTGGT | 20 |

**[Table3]**

| | Fibrotic marker primer sequences | | | |
|---|---|---|---|---|
| | Sense | SEQ ID NO: | Anti-sense | SEQ ID NO: |
| Col1α | F-ACAGTCCAGTTCTTCATTGC | 21 | R-GCACTCTTCTCCTGGTCCTG | 22 |
| α-SMA | F-CTGACAGAGGCACCACTGAA | 31 | R-CATCTCCAGAGTCCAGCACA | 32 |
| mGAPDH | F-CCGTATTGGGCGCCTGGTCA | 33 | R-CCGGCCTTCTCCATGGTGGT | 34 |

### 1-6) Electrophysiological analysis

Whole cell current through the cell membrane in isolated and cultured single fibroblasts was measured using a patch-clamp technique. A voltage ramp was used to apply voltage to whole-cell voltage clamp cells from -100 mV to +100 mV using a micro-glass electrode, the generated current was amplified using an amplifier (EPC-10, HEKA, Lambrecht, Germany) and then recorded at a sampling rate of 1 to 4 kHz.

A standard external solution contained 150 mM NaCl, 6 mM KCl, 1.5 mM CaCl₂, 1 mM MgCl₂, 10 mM HEPES, and 10 mM glucose at pH 7.4 (adjusted with NaOH), and the micro glass electrode (pipette) solution contained 40 mM KCl, 100 mM K-aspartate, 2 mM MgCl₂, 0.1 mM EGTA, 4 mM Na₂ATP, and 10 mM HEPES at pH 7.2 (adjusted with KOH). The free Ca²⁺ concentration in the pipette solution was adjusted to 1 µM by adding an appropriate amount of Ca²⁺ in the presence of 5 mM EGTA (calculated with CaBuf; G. Droogmans, Leuven, Belgium).

K_{Ca}3.1 current was isolated using the following method. In a current recorded by injecting 1 µM Ca²⁺ into whole-cell voltage clamp cells using a glass electrode and applying 1-ethyl-2-benzimidazolinone (1-EBIO, 100 µM) activating K_{Ca}3.1 current, a current inhibited by K_{Ca}3.1 channel inhibitor TRAM-34 (10 µM) was determined as the K_{Ca}3.1 current, and the recorded current was normalized by division by cell capacitance.

### 1-7) Statistical analysis

Experimental results were expressed as mean ± standard error of the mean (S.E.M). Statistical analysis was performed by the Student's t-test, and a significance level of 0.05 or less was determined as significantly different.

### 2) Experimental results using cultured cells

### 2-1) Effect of SF-2 compound on K_{Ca}3.1 current

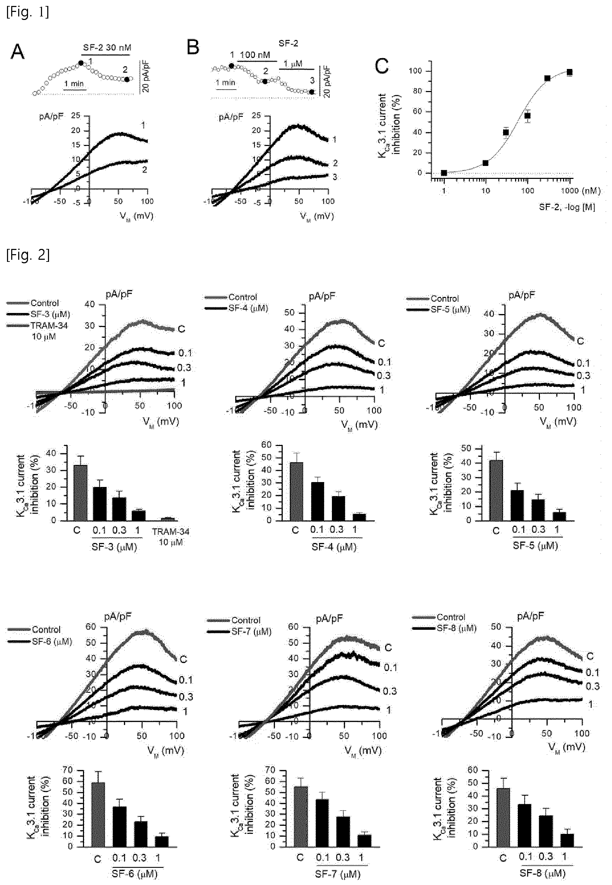

FIG. 1 shows the effect of the SF-2 compound on K_{Ca}3.1 channel current in the fibroblasts. The K_{Ca}3.1 current activated by Ca²⁺ and 1-EBIO in cells was inhibited by SF-2 in a concentration-dependent manner.

The size of the K_{Ca}3.1 current was 37.49±1.51 pA/pF in cells not exposed to the SF-2 compound, and the size of the K_{Ca}3.1 current was significantly reduced to 26.65±1.89 mV/pF, 11.69±1.66 mV/pF, 7.12±1.33 mV/pF, 1.87±0.24 mV/pF, and 1.95±0.44 mV/pF in cells exposed to 10, 30, 100, 300, and 1000 nM of the SF-2 compound, respectively.

### 2-2) Effect of SF-3 to SF-8 compounds on K_{Ca}3.1 current

FIG. 2 shows the effects of SF-3 to SF-8 compounds on the K_{Ca}3.1 channel current in the fibroblasts. The K_{Ca}3.1 current activated by Ca²⁺ and 1-EBIO in cells was inhibited by the SF-3 to SF-8 compounds in a concentration-dependent manner.

### 2-3) Inflammation inhibitory effect by SF-3 to SF-8 compounds

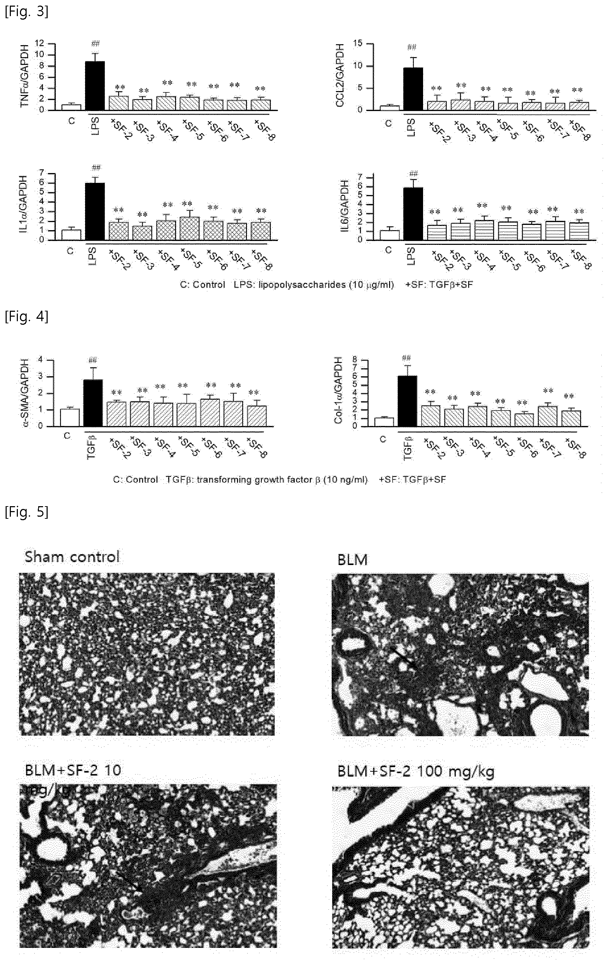

FIG. 3 shows the inhibitory effect of SF-2 to SF-8 compounds on inflammation induced by LPS in the fibroblasts. The inflammation-inducing effect by LPS was determined by the degree of mRNA expression of an inflammatory marker. When the fibroblasts were exposed to LPS (10 µg/ml) for 24 hours, an mRNA level of IL6, which is an inflammatory marker, increased.

In addition, when the fibroblasts were exposed to one of the SF-2 to SF-8 compounds together with LPS for 24 hours, the degree of mRNA expression of the inflammatory marker decreased. In FIG. 3, the experimental results are expressed as mean ± SE, and n=6~8, ## < 0.01 (normal control vs. disease-induced group) and ^{∗∗} < 0.01 (disease-induced group vs. drug-administered group).

### 2-4) Fibrosis inhibitory effect by SF-3 to SF-8 compounds

FIG. 4 shows the fibrosis inhibitory effects of the SF-2 to SF-8 compounds on fibrosis caused by TGF_{β} in fibroblasts. Here, the fibrosis-inducing effect by TGF_{β} was determined by the degree of mRNA expression of a fibrotic marker. When the fibroblasts are exposed to TGF_{β} (10 ng/ml) for 24 hours, the mRNA levels of the fibrotic markers, α-smooth muscle actin (α-SMA), collagen 1α (Col1α), and collagen 3α (Col3α), increased.

In addition, when the fibroblasts are exposed to one of the SF-2 to SF-8 compounds together with TGF_{β} for 24 hours, the expression degrees of the fibrotic markers decreased. In FIG. 4, the experimental results are expressed as mean ± SE, and n=6, ## < 0.01 (normal control vs. disease-induced group) and ^{∗∗} < 0.01(disease-induced group vs. drug-administered group).

### 3) Effect of SF-2 compound on lung disease mouse models

### 3-1) Histological and immunohistological analysis results

Paraffinized tissue specimens were prepared using lung tissue extracted from the mouse models, and immunohistological staining for the fibrotic marker, collagen, was performed. FIG. 5 shows the Masson's trichrome staining result of the lung tissue, and it was confirmed that a fibrosis degree in a disease-induced group (BLM), compared to a normal control (Sham control), increased, and the fibrosis degree decreased in a drug-administered group (BLM+SF-2).

Accordingly, it can be confirmed that the SF-2 compound of the present invention has efficacy of inhibiting lung fibrosis. In FIG. 5, 'BLM' is a group with a disease induced by treatment of 1.5 units of bleomycin, and 'BLM+SF-2' is a drug-administered group to which the SF-2 compound was administered at 10 mg/kg/day or 100 mg/kg/day, together with 1.5 units of bleomycin.

### 3-2) Analysis result for fibrotic marker mRNA expression

To confirm the effect of the SF-2 compound on the expression of fibrotic marker mRNA in the mouse models, RT-PCR was performed for fibrotic markers Col1α (collagen 1α), Col3α (collagen 3α) and α-SMA (α-smooth muscle actin).

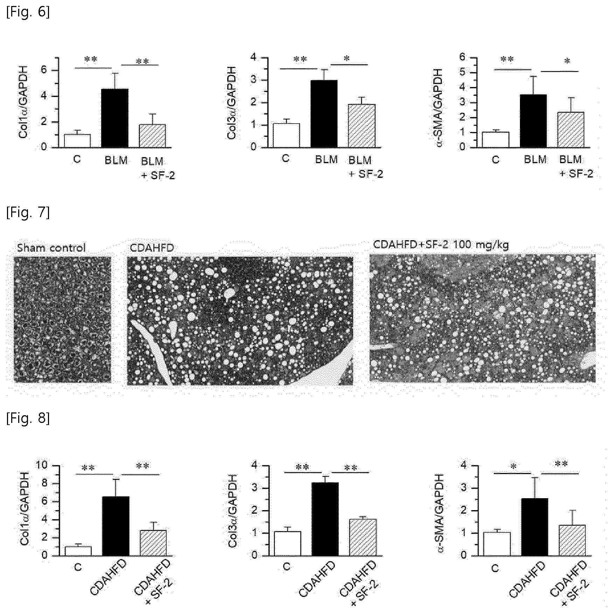

As shown in FIG. 6, mRNA expression levels of Col1α, Col3α and α-SMA were greatly increased in lung tissue of a disease-induced group (BLM), compared to a normal control (C), indicating that fibrosis had progressed. However, it was confirmed that, in the drug-administered group (BLM+SF-2), the mRNA expression levels of Col1α, Col3α and α-SMA greatly decreased and thus fibrosis was inhibited. This result suggests that the SF-2 compound of the present invention has an effect of inhibiting lung fibrosis. In FIG. 6, the experimental results are expressed as mean ± SE, and n=6, * < 0.05 and ** < 0.01.

### 4) Experimental results for liver disease mouse models

### 4-1) Histological and immunohistological analyses

FIG. 7 shows the result of Masson's trichrome staining for the collagen fiber of the liver tissue, and confirmed that the liver tissue of a normal control (Sham control) is in a healthy state with no fibrosis yet, but the liver tissue of a disease-induced group (CDAHFD) is stained blue, indicating that fibrosis is progressing. However, it was observed that the degree of blue staining of the liver tissue of the drug-administered group (CDAHFD+SF-2) prepared by administering the SF-2 compound to the disease-induced group greatly decreased and fibrosis was greatly inhibited.

### 4-2) Analysis result for expression of fibrotic marker mRNA

To confirm an effect of the SF-2 compound on the expression of fibrotic marker mRNA in the mouse models, RT-PCR was performed for fibrotic markers Col1α (collagen 1α), Col3α (collagen 3α) and α-SMA (α-smooth muscle actin).

As shown in FIG. 8, the mRNA expression levels of Col1α, Col3α and α-SMA greatly increased, indicating that fibrosis had progressed in the lung tissue of the disease-induced group (CDAHFD), compared to the normal control (C). However, it was confirmed that, in the drug-administered group (CDAHFD+SF-2), the mRNA expression levels of Col1α, Col3α and α-SMA greatly decreased and fibrosis was inhibited.

This result suggests that the SF-2 compound of the present invention has an effect of inhibiting liver fibrosis. In FIG. 8, the experimental results are expressed as mean ± SE, and n=6, ^{∗} < 0.05 and ^{∗∗} < 0.01.

### 5) Evaluation of experimental results

From the above experimental results, it was confirmed that the compound of Chemical Formula 2 of the present invention, i.e., solriamfetol has efficacy of inhibiting fibrosis in a mouse model in which lung fibrosis is induced by bleomycin and a mouse model in which liver fibrosis is induced by a CDAHFD diet.

In addition, the compounds of Chemical Formulas 2 to 8 of the present invention have efficacy of inhibiting inflammation and fibrosis in fibroblasts in which inflammation is induced by LPS and fibroblasts in which fibrosis is induced by TGF_{β}, respectively. This result shows that the compounds of Chemical Formulas 2 to 8 have an effect of inhibiting K_{Ca}3.1 channel expression.

Meanwhile, as described in the background, the K_{Ca}3.1 channel plays an important role in the progression of autoimmune diseases and cancer diseases, as well as inflammatory and fibrotic diseases. Therefore, several research groups or pharmaceutical companies around the world are developing therapeutic agents for autoimmune diseases, inflammatory and fibrotic diseases, and cancer diseases using K_{Ca}3.1 channel inhibitors such as TRAM-34 and senicapoc.

As such, when the K_{Ca}3.1 channel is inhibited, it can inhibit the progression of immune diseases and cancer diseases, as well as inflammatory and fibrotic diseases, and thus it is possible to conclude that the compounds of Chemical Formulas 2 to 8 of the present invention are also effective in treatment of autoimmune diseases and cancer diseases.

Meanwhile, in the present invention, due to practical limitations, the above experiments were not conducted for all compounds belonging to the compound of Chemical Formula 1, but considering chemical activity and metabolic mechanisms *in vivo*, it is assumed that all of the compounds of Chemical Formula 1 have pharmacological efficacy similar to the compounds of Chemical Formulas 2 to 8.

## Claims

1. A composition for treating a K_{Ca}3.1 channel-mediated disease, comprising:
a phenylalkyl carbamate compound represented by Chemical Formula 1 below or a pharmaceutically acceptable salt thereof: 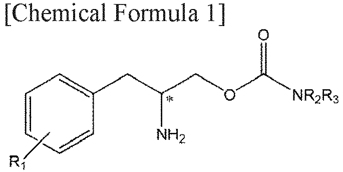 In Chemical Formula 1, R₁ is one or two functional groups selected from hydrogen, halogen, hydroxyl, amine, nitro, hydrogen sulfide, methyl, methylhalogen, ethyl, propyl, methoxy, ethoxy, vinyl, and aryl, each of R₂ and R₃ is one functional group selected from hydrogen, methyl, ethyl, propyl, and amide, and * indicates a chiral center.

2. The composition of claim 1, wherein R₂ and R₃ are hydrogen in Chemical Formula 1.

3. The composition of claim 1, wherein, in Chemical Formula 1, R₁ is one or two functional groups selected from hydrogen, F, Cl, Br, and I.

4. The composition of claim 1, wherein, in Chemical Formula 1, R₁ is one or two Fs, and each of R₂ and R₃ is one selected from hydrogen, methyl and amide.

5. The composition of claim 1, wherein the compound represented by Chemical Formula 1 is a chiral compound having an R-isomer or S-isomer content of 90% or more.

6. The composition of claim 1, wherein the compound represented by Chemical Formula 1 is any one selected from 2-amino-3-phenylpropylcarbamate; 2-amino-3-(3-fluorophenyl)propylcarbamate; 2-amino-3-(3,4-dichlorophenyl)propylcarbamate; 2-amino-3-phenylpropylmethylcarbamate; 2-amino-3-phenylpropyl(aminocarbonyl)carbamate; 2-amino-3-(4-hydroxylphenyl)propylcarbamate; and 2-amino-3-[3-(trifluoromethyl)phenyl]propylcarbamate.

7. The composition of claim 1, wherein the compound represented by Chemical Formula 1 is (R)-2-amino-3-phenylpropylcarbamate hydrochloride.

8. The composition of any one of claims 1 to 7, wherein the K_{Ca}3.1 channel-mediated disease is a fibrotic disease such as liver fibrosis or lung fibrosis.

9. The composition of any one of claims 1 to 7, wherein the K_{Ca}3.1 channel-mediated disease is an autoimmune disease.

10. The composition of any one of claims 1 to 7, wherein the K_{Ca}3.1 channel-mediated disease is a cancer disease.
